# EUROPEAN PATENT APPLICATION

(11) **EP 3 922 309 A1**
(43) Date of publication of application: **15.12.2021**
(21) Application number: 20382508.8
(22) Date of filing: 12.06.2020
(51) Int. Cl.: A61P 1/00, A23K 10/18, A23L 33/135, A61K 35/747, A61P 31/04, C12N 1/20, C12R 1/225

(54) **NOVEL LACTOCOCCUS LACTIS STRAIN FOR THE PRODUCTION OF BIOACTIVE COMPOUNDS HAVING ANTIMICROBIAL EFFECT**

(71) Applicant: Innovación en Gestón y Conservación de Ungulados S.L. (Ingulados), 10004 Cáceres (ES)
(72) Inventor: FERNÁNDEZ LLARIO, Pedro, 10004 Cáceres (ES); CERRATO HORRILLO, Rosario, 10004 Cáceres (ES); GARCIA JIMÉNEZ, Waldo L., 10004 Cáceres (ES); GONÇALVES BLANCO, Pilar, 10004 Cáceres (ES); BRAVO SANTILLANA, María, 10004 Cáceres (ES); ARENAS MANZANO, Verónica, 10004 Cáceres (ES); MORENO REY, María Eugenia, 10004 Cáceres (ES); RISCO PEREZ, David, 10004 Cáceres (ES)
(74) Representative: Clarke Modet & Co.

(57) **Abstract**

Disclosed herein is a novel Lactococcus lactis strain, termed as C11JN and with deposit number DSM 33521 and antimicrobial compositions comprising said strain and/or bioactive compounds produced by said strain. This new strain and the bioactive compounds that it produces have shown to be useful to inhibit the growth of a wide-spectrum bacterial pathogens and, hence, to treat diseases produced by said bacterial pathogens.

## Description

### FIELD OF THE INVENTION

The present invention is related to the food, pharmaceutical and veterinary industries, more specifically to food, pharmaceutical or veterinary compositions comprising bioactive compounds obtained from a specific *Lactococcus lactis* strain and which are used for the treatment of several bacterial infections.

### BACKGROUND ART

Antibiotic resistance happens when germs like bacteria and fungi develop the ability to defeat the drugs designed to kill them. That means the germs are not killed and continue to grow. Antibiotic resistance does not mean the body is becoming resistant to antibiotics; it is that bacteria have become resistant to the antibiotics designed to kill them.

Infections caused by antibiotic-resistant germs are difficult, and sometimes impossible, to treat. In most cases, antibiotic-resistant infections require extended hospital stays, additional follow-up doctor visits, and costly and toxic alternatives.

Antibiotic resistance has the potential to affect people at any stage of life, as well as the healthcare, veterinary, and agriculture industries, making it one of the world's most urgent public health problems.

United States Patent application US2014154217A1 discloses *Lactococcus lactis* strains, NRRL B-50571 and NRRL B-50572, and a bacterial preparation containing the same, have the ability to produce bioactive peptides that reduce blood pressure, lower LDL-cholesterol (bad cholesterol) and present antioxidant properties for better cardiovascular health. These biologically active peptides may be produced within the food for the production of a food product, such as a functional food, or they may be produced from protein sources and subsequently added to a food as part of the formulation or as part of a food supplement or a pharmaceutical preparation.

PCT Patent application WO2019178325A1 relates to methods of using beneficial bacterial such as, a *Lactococcus lactis* strain, to manage, treat, or prevent obesity, fatty liver disease, harmful ionizing radiation, and other conditions or aliments. In certain embodiments, the subject is at risk or, susceptible to, exhibiting symptoms of, or diagnosed with a disease or conditions as mentioned above. In certain embodiments, methods comprise administering an effective amount of a *Lactococcus lactis* strain to a subject in need thereof. In certain embodiments, the *Lactococcus lactis* strain is *Lactococcus lactis* subsp. cremoris ATCC^{®} 19257 or a mutant thereof.

United States Patent application US2014093487A1 describes an antimicrobial composition comprising a *Lactobacillus rhamnosus strain* and/or a *Lactobacillus paracasei* strain. Furthermore, said document discloses uses for such an antimicrobial composition, food, feed and pharmaceutical products comprising such an antimicrobial composition, a method of manufacturing such food, feed and pharmaceutical products and a method for reducing the content of unwanted microorganisms of such food, feed and pharmaceutical products.

Nevertheless, and given the growing problem with antibiotic resistance, there is the need for additional compositions with antimicrobial (preferably, antibacterial) activity which can aid in the management of said antibiotic resistance.

The inventors of the present invention, after extensive and exhaustive research, have surprisingly found a *Lactococcus lactis* strain which is able to produce bioactive compounds which are effective against a broad range of pathogen bacteria. Due to the activity that said compounds have, they can be used in substitution of current treatments or in conjunction with them to improve the outcome in the treatment of a broad spectrum of pathogen bacteria, even in the case of bacteria with resistance to current antibiotics (as these are new compounds against which bacteria do not have yet resistance), both in humans and in animals.

Therefore, the present invention solves the technical problems present in the state of the art and mentioned above.

### SUMMARY OF INVENTION

Therefore, in a first aspect, the present invention refers to a strain of *Lactococcus lactis* termed *Lactococcus lactis* C11JN (Deposit number: DSM 33521).

In a second aspect, the present invention refers to an antimicrobial composition comprising *Lactococcus lactis* C11JN (Deposit number DSM 33521), one or more compounds obtained from the strain *Lactococcus lactis* C11JN (Deposit number DSM 33521) or combinations thereof.

In an additional aspect, the present invention refers to a food composition that comprises the strain *Lactococcus lactis* C11JN (Deposit number: DSM 33521) of the present invention, an antimicrobial composition of the present invention or combinations thereof.

In a fourth aspect, the present invention refers to a pharmaceutical composition comprising the strain *Lactococcus lactis* C11JN (Deposit number: DSM 33521) of the present invention, an antimicrobial composition of the present invention or combinations thereof.

Furthermore, the present invention also refers to a veterinary composition comprising the strain *Lactococcus lactis* C11JN (Deposit number: DSM 33521) of the present invention, an antimicrobial composition of the present invention or combinations thereof.

In a sixth aspect, the present invention refers to a probiotic composition comprising the strain *Lactococcus lactis* C11JN (Deposit number: DSM 33521) of the present invention.

In a seventh aspect, the present invention refers to a postbiotic composition comprising one or more compounds obtained from the strain *Lactococcus lactis* C11JN (Deposit number DSM 33521) or a combinations of the strain *Lactococcus lactis* C11JN (Deposit number: DSM 33521) of the present invention and one or more compounds obtained from the strain Lactococcus lactis C11JN (Deposit number DSM 33521).

In an eighth aspect, the present invention refers to a *Lactococcus lactis* C11JN (Deposit number: DSM 33521) of the present invention, an antimicrobial composition of the present invention, a pharmaceutical composition of the present invention or a veterinary composition of the present invention for use as a medicament.

In an ninth aspect, the present invention refers to a *Lactococcus lactis* C11JN (Deposit number: DSM 33521) of the present invention, an antimicrobial composition of the present invention, a pharmaceutical composition of the present invention or a veterinary composition of the present invention for use in the prevention, amelioration or treatment of a microbial disease.

In a tenth aspect, the present invention refers to the use of *Lactococcus lactis* C11JN (Deposit number: DSM 33521) of the present invention, an antimicrobial composition of the present invention, a pharmaceutical composition of the present invention or a veterinary composition of the present invention for the manufacture of a medicament for the prevention, amelioration or treatment of a microbial disease.

In a final aspect, the present invention refers to a method for the prevention, amelioration or treatment of a microbial disease comprising administering *Lactococcus lactis* C11JN (Deposit number: DSM 33521) of the present invention, an antimicrobial composition of the present invention, a pharmaceutical composition of the present invention or a veterinary composition of the present invention to a subject in need thereof

As used herein, the term "probiotic composition", "probiotic" and their plurals have the meaning commonly attributed in the state of the art, this is, a composition comprising a live microorganism, which when administered in adequate amounts confers a health benefit to the subject receiving the probiotic composition.

As used herein, the term "postbiotic composition", "postbiotic" and their plurals have the meaning commonly attributed in the state of the art, this is, a composition comprising functional bioactive compounds generated in a matrix during a fermentation carried out by a bacteria and which may be used to promote health of a subject receiving said postbiotic composition.

As used herein, the term "symbiotic" and its plural have the meaning they commonly acquire in the state of the art, this is, they refer to mixtures of probiotics and prebiotics that beneficially affect the subject receiving them by improving the survival and colonization of live beneficial microorganism in the gastrointestinal tract of said subject.

As used herein "prebiotic" and its plural have the meaning they commonly acquire in the state of the art, this is, they refer to substrate/s that is/are selectively utilized by microorganisms in the subject receiving the prebiotic conferring a health benefit.

"*Lactobacillus lactis* C11JN", "*Lactobacillus lactis* C11JN (Deposit number DSM 33521)", "strain *Lactobacillus lactis* C11JN (Deposit number DSM 33521)", "*Lactobacillus lactis* C11JN strain", "DSM 33521" and "C11JN" are used interchangeably herein and have the same meaning as they refer to the bacterial strain *Lactococcus lactis* C11JN and deposited in the Leibniz Institute DSMZ-German Collection of Microorganisms and Cell Cultures with Deposit number DSM 33521.

As used in the present document, "De Mann Rogosa and Sharpe", "De Mann Rogosa and Sharpe culture medium", "MRS culture medium", "De Mann Rogosa and Sharpe medium", "MRS medium", "MRS" and their plurals are used interchangeably and refer to a culture medium generally known in the state of the art. As used herein, this medium when it is in the form of agar has the following composition: 20 g/L of dextrose, 10 g/L of bacteriologic peptone, 8 g/L of beef extract, 5 g/L of sodium acetate, 5 g/L of yeast extract, 2 g/L of dipotassium phosphate, 1 g/L of Tween 80, 2 g/L of triammonium citrate, 0.2 g/L of magnesium sulfate, 0.05 g/L of manganese sulfate and 10 g/L of agar. As used herein, this medium when it is in the form of broth has the following composition: 20 g/L of dextrose, 10 g/L of bacteriologic peptone, 8 g/L of beef extract, 5 g/L of sodium acetate, 5 g/L of yeast extract, 2 g/L of dipotassium phosphate, 1 g/L of Tween 80, 2 g/L of triammonium citrate, 0.2 g/L of magnesium sulfate and 0.05 g/L of manganese sulfate.

As used in the present document, "Mueller-Hinton", "Mueller-Hinton culture medium", "Mueller-Hinton medium", "MH culture medium", "MH medium", "MH" and their plurals, are used interchangeably and refer to a culture medium generally known in the state of the art. As used herein, this medium when it is in the form of agar has the following composition: 17.5 g/L of casein acid peptone, 2 g/L of beef infusion, 1.5 g/L of starch and 17 g/L of agar. As used herein, this medium when it is in the form of broth has the following composition: 17.5 g/L of casein acid peptone, 2 g/L of beef infusion and 1.5 g/L of starch.

As used in the present document, "Trypton Soy Broth", "TSB", "and their plurals are used interchangeably and refer to a culture medium generally known in the state of the art. As used herein, this medium when it is in the form of broth has the following composition: 17 g/L of casein peptone, 3 g/L of soy peptone, 5 g/L of Sodium chloride, 2.5 g/L of phosphate dipotassium, 2.5 g/L of dextrose.

### DETAILED DESCRIPTION

As noted above, in a first aspect, the present invention refers to a strain of *Lactococcus lactis* termed *Lactococcus lactis* C11JN and deposited in the Leibniz Institute DSMZ-German Collection of Microorganisms and Cell Cultures with Deposit number DSM 33521.

This new and inventive bacterial strain, as noted in the examples, has been isolated from the nasal mucosa of wild boar.

In addition, it has been observed that the *Lactococcus lactis* C11JN strain is a Gram-positive coccus and catalase negative. As coccus, *Lactococcus lactis* C11JN strain cells are spherical or ovoid and occur singly, in pairs, or in chains, and are often elongated in the direction of the chain.

Further features of *Lactococcus lactis* C11JN strain are:
- Cells do not form endospores.
- Cells are nonmotile.
- Cells are not β-hemolytic.
- Cells are aerobic.

Furthermore, the genomic deoxyribonucleic acid (hereinafter, DNA) sequence of *Lactococcus lactis* C11JN is SEQ ID NO: 1.

Moreover, within its genomic DNA sequence, *Lactococcus lactis* C11JN it seems to comprises sequences codifying for:
- Nisins:
   - Nisin A (SEQ ID NO: 3), which would correspond to positions 1864240 to 1864341 of SEQ ID NO: 1 (SEQ ID NO: 2);
   - Nisin Z (SEQ ID NO: 4), which would correspond to positions 1864240 to 1864341 of SEQ ID NO: 1 (SEQ ID NO: 2);
   - Nisin Q (SEQ ID NO: 5), which would correspond to positions 1864240 to 1864341 of SEQ ID NO: 1 (SEQ ID NO: 2);
   - Nisin F (SEQ ID NO: 6), which would correspond to positions 1864240 to 1864341 of SEQ ID NO: 1 (SEQ ID NO: 2);
   - Nisin U (SEQ ID NO: 8), which would correspond to positions 1864240 to 1864329 of SEQ ID NO: 1 (SEQ ID NO: 7).
- Enterolysin A (SEQ ID NO: 10), which would correspond to positions 29295 to 29723 of SEQ ID NO: 1 (SEQ ID NO: 9).
- Subtilin (SEQ ID NO: 12), which would correspond to positions 1864246 to 1864329 of SEQ ID NO: 1 (SEQ ID NO: 11).
- Zoocin A (SEQ ID NO: 14), which would correspond to positions 613895 to 614665 of SEQ ID NO: 1 (SEQ ID NO: 13).

In addition, the strain of the present invention can be preferably cultured in Mann Rogosa and Sharpe (MRS) culture medium or Tryptone Soy Broth (TSB), under aerobiosis conditions and at a temperature of between 30 °C to 37 °C. More preferably, the strain of the present invention is cultured in MRS culture medium at 30 °C under aerobiosis conditions.

Moreover, cells of *Lactococcus lactis* C11JN strain have a fermentative metabolism and their predominant end product of glucose, ribose, arabinose, lactose and trehalose fermentation is lactic acid.

As already noted above, and as can be deducted from the examples included below, this new and inventive strain has a wide-spectrum activity against bacteria (antibiotic activity), including both, gram-positive and gram-negative bacteria. The bacterial strain of the present invention has been shown to:
- Inhibit growth of, at least: *Listeria monocytogenes, Streptococcus suis, Enterococcus faecium, Salmonella typhimurium, Escherichia coli, Manheimia haemolytica 813, Pasterurella spp. 1315,2, Pasteurella multocida A* 1269,2, *Pasteurella multocida B 1291,1, Pasteurella multocida D 1277,4, Biberstenia trehalosi 1315,1 or Clostridium perfringens* (with surprisingly high activity against *Pasterurella spp. 1315,2, Pasteurella multocida A 1269,2, Pasteurella multocida B 1291,1, Pasteurella multocida D 1277,4, Biberstenia trehalosi 1315,1).*
- Produce a wide variety of bacteriocins.
- Produce wide variety of bioactive compounds active against a wide variety of bacteria.

Therefore, the *Lactococcus lactis* C11JN strain of the present invention solves the problems present in the state of the art and mentioned above providing a new treatment against bacterial pathogens, allowing to overcome current antibiotic resistance. This new treatment can be used alone or in combination with current therapies to improve their activities, to treat a broad-spectrum of bacterial diseases, both in animals and humans (preferably, in animals).

In a second aspect, the present invention provides for an antimicrobial composition comprising *Lactococcus lactis* C11JN (Deposit number DSM 33521), one or more compounds obtained from the strain *Lactococcus lactis* C11JN (Deposit number DSM 33521) or combinations thereof.

*Lactococcus lactis* C11JN (Deposit number DSM 33521) is as already described above in the first aspect of the present invention.

In a most preferred embodiment, the one or more compounds obtained from the strain *Lactococcus lactis* C11JN (Deposit number DSM 33521) are, preferably, the fermented medium of strain *Lactococcus lactis* C11JN (Deposit number DSM 33521) of the present invention; more preferably, the culture supernatant of a culture of the strain *Lactococcus lactis* C11JN (Deposit number DSM 33521); even more preferably, the culture supernatant of a culture of the strain *Lactococcus lactis* C11JN (Deposit number DSM 33521) together with the *Lactococcus lactis* C11JN (Deposit number DSM 33521) cells inactivated.

It is also contemplated that the one or more compounds obtained from the strain Lactococcus lactis C11JN (Deposit number DSM 33521) are one or more bioactive compounds secreted by the strain *Lactococcus lactis* C11JN (Deposit number DSM 33521) of the present invention; more preferably one or more bioactive compounds with antimicrobial activity secreted by the strain Lactococcus lactis C11JN (Deposit number DSM 33521) of the present invention; even more preferably at least one type of Nisin, Enterolysin A, Subtilin, Zoocin A or combinations thereof, secreted by the strain Lactococcus lactis C11JN (Deposit number DSM 33521) of the present invention; even more preferably Nisin A, Nisin Z, Nisin Q, Nisin F, Nisin U, Enterolysin A, Subtilin, Zoocin A or combinations thereof, secreted by the strain Lactococcus lactis C11JN (Deposit number DSM 33521) of the present invention; still more preferably Nisin A, Nisin Z, Nisin Q, Nisin F, Nisin U, Enterolysin A, Subtilin and Zoocin A secreted by the strain Lactococcus lactis C11JN (Deposit number DSM 33521) of the present invention.

Nisin A, preferably, comprises SEQ ID NO: 3; more preferably, Nisin A consists of SEQ ID NO: 3.

Nisin Z, preferably, comprises SEQ ID NO: 4; more preferably, Nisin Z consists of SEQ ID NO: 4.

Nisin Q, preferably, comprises SEQ ID NO: 5; more preferably, Nisin Q consists of SEQ ID NO: 5.

Nisin F, preferably, comprises SEQ ID NO: 6; more preferably, Nisin F consists of SEQ ID NO: 6.

Nisin U, preferably, comprises SEQ ID NO: 8; more preferably, Nisin U consists of SEQ ID NO: 8.

Enterolysin A, preferably, comprises SEQ ID NO: 10; more preferably, Enterolysin A consists of SEQ ID NO: 10.

Subtilin, preferably, comprises SEQ ID NO: 12; more preferably, Subtilin consists of SEQ ID NO: 12.

Zoocin A, preferably, comprises SEQ ID NO: 14; more preferably, Zoocin A consists of SEQ ID NO: 14.

It is contemplated that the antimicrobial composition comprises one or more further compounds. Said one or more further compounds can be any compound known in the state of the art.

In a preferred embodiment, the antimicrobial composition of the present invention comprises *Lactococcus lactis* C11JN (Deposit number DSM 33521) but not one or more compounds obtained from the strain *Lactococcus lactis* C11JN (Deposit number DSM 33521), more preferably, in this preferred embodiment, the antimicrobial composition only comprises as active ingredient *Lactococcus lactis* C11JN (Deposit number DSM 33521).

In another preferred embodiment, the antimicrobial composition comprises one or more compounds obtained from the strain Lactococcus lactis C11JN (Deposit number DSM 33521) but not *Lactococcus lactis* C11JN (Deposit number DSM 33521), more preferably in this preferred embodiment, the antimicrobial composition only comprises as active ingredient the one or more compounds obtained from the strain *Lactococcus lactis* C11JN (Deposit number DSM 33521).

In the antimicrobial composition of the present invention, the *Lactococcus lactis* C11JN (Deposit number DSM 33521) can be produced by any means known in the art. Preferably, the Lactococcus lactis C11JN (Deposit number DSM 33521) of the present invention is produced by means of a process comprising the steps of a) culturing Lactococcus lactis C11JN (Deposit number DSM 33521); and b) inactivating the culture of Lactococcus lactis C11JN (Deposit number DSM 33521) obtained in step a). More preferably, the step a) of culturing comprises culturing the *Lactococcus lactis* C11JN (Deposit number DSM 33521) of the present invention in a suitable culture medium, at a temperature between 30 °C and 37 °C for between 12 and 36 hours under aerobiosis conditions; still more preferably the step a) of culturing comprises culturing the Lactococcus lactis C11JN (Deposit number DSM 33521) of the present invention in De Mann Rogosa and Sharpe culture medium (MRS culture medium) at 30 °C during 24h in aerobiosis conditions. Also, preferably in the inactivation step, *Lactococcus lactis* C11JN (Deposit number DSM 33521) obtained in step a) are heated at between 80 °C and 100 °C during between 30 min and 4 hours; more preferably at 80°C during 1 hour.

In addition, the one or more compounds obtained from the strain *Lactococcus lactis* C11JN (Deposit number DSM 33521) can be produced by any means known in the art. Preferably, they are obtained by means of a procedure comprising the following steps:
a) collecting the culture supernatant of a culture of *Lactococcus lactis* C11JN (Deposit number DSM 33521) of the present invention;
b) centrifuging said supernatant to obtain a centrifuged supernatant, preferably 5-15 minutes at 5,000-7,000 rpm, more preferably 10 minutes at 6,000 rpm;
c) filtering the centrifuged supernatant, preferably through a 0.22 µm filter;
d) inactivating the solution obtained in step c) by means of heat treatment, preferably by means of thermoblock 8-10 minutes at 100 °C.

The *Lactococcus lactis* C11JN (Deposit number DSM 33521) of the present invention, before step a) mentioned above can be cultured under any suitable condition and as explained above. More preferably, before step a) *Lactococcus lactis* C11JN (Deposit number DSM 33521) is cultured in aerobiosis conditions, in De Mann Rogosa and Sharpe culture medium and at 30 °C, for 24 hours and under aerobiosis conditions. The latter are the optimal culture conditions for the strain of the present invention for the production and secretion of bioactive compounds with antimicrobial activity (activity against bacterial pathogens).

The one or more bioactive compounds secreted by the strain *Lactococcus lactis* C11JN (Deposit number DSM 33521) of the present invention (more preferably one or more bioactive compounds with antimicrobial activity secreted by the strain *Lactococcus lactis* C11JN (Deposit number DSM 33521) of the present invention; even more preferably Nisin A, Nisin Z, Nisin Q, Nisin F, Nisin U, Enterolysin A, Subtilin, Zoocin A or combinations thereof, secreted by the strain *Lactococcus lactis* C11JN (Deposit number DSM 33521) of the present invention) mentioned above, can be produced by any means known in the state of the art. Preferably, they are purified from the fermented medium mentioned above, more preferably the purification is performed by means of ultrafiltration selecting the protein fraction of between 3 and 10 kDa. However, it is also contemplated and within the scope of protection of the present Patent application the same bioactive compounds being produced by means of chemical synthesis.

The antimicrobial composition of the present invention can comprise any excipient known in the art, which is suitable for the intended use and which does not alter or modify in an unacceptable manner the activity of the antimicrobial composition.

It is contemplated that antimicrobial composition of the present invention has any form known in the state of the art which is suitable for the contemplated or desired administration route (more preferably, for oral administration). Preferably, the antimicrobial composition of the present invention is in solid or liquid form, more preferably in the form of a tablet, powder, solid lyophilized or liquid, more preferably solid lyophilized or liquid.

As it is directly derivable from the examples included below, the antimicrobial composition of the present invention has a broad-spectrum activity against bacteria (antibiotic activity), including both, gram-positive and gram-negative bacteria. Therefore, provides a new treatment against bacterial pathogens, allowing to overcome current antibiotic resistance. The antimicrobial composition of the present invention can be used alone or in combination with current therapies to improve their activities, to treat a broad-spectrum of bacterial diseases, both in animals and humans (preferably, in animals).

In third aspect, as already noted above, the present invention refers to a food composition that comprises *Lactococcus lactis* C11JN (Deposit number: DSM 33521) of the present invention, an antimicrobial composition of the present invention or combinations thereof.

*Lactococcus lactis* C11JN (Deposit number: DSM 33521) is as explained above in the first aspect of the present invention.

The antimicrobial composition of the present invention is as explained above in the second aspect of the present invention.

The food composition can be in the form of any suitable food known in the state of the art.

In a preferred embodiment, the food composition of the present invention is a food supplement, a nutraceutical, a probiotic, a symbiotic or a postbiotic, more preferably a probiotic or a postbiotic.

In a fourth aspect, the present invention refers to a pharmaceutical composition comprising *Lactococcus lactis* C11JN (Deposit number: DSM 33521) of the present invention, an antimicrobial composition of the present invention or combinations thereof.

*Lactococcus lactis* C11JN (Deposit number: DSM 33521) is as explained above in the first aspect of the present invention.

The antimicrobial composition of the present invention is as explained above in the second aspect of the present invention.

The pharmaceutical composition of the present invention can be in any form known in the state of the art, preferably, the pharmaceutical composition of the present invention is in solid or liquid form, more preferably in the form of a tablet, powder, solid lyophilized or liquid; more preferably, tablet, powder or solid lyophilized; even more preferably, solid lyophilized.

Preferably, the pharmaceutical composition of the present invention is adapted or suitable for oral administration.

It is contemplated that the pharmaceutical composition of the present invention comprises one or more further active compounds. Said one or more further active compounds can be any compound known in the state of the art. Preferably, said one or more active compounds are selected from antibiotics, more preferably β-lactam antibiotics, more preferably amoxicillin, cephalosporin or combinations thereof.

It is contemplated that the pharmaceutical composition of the present invention comprises any suitable pharmaceutical excipient known in the state of the art. Said excipients must not unacceptably alter the activity of the strain *Lactococcus lactis* C11JN (Deposit number: DSM 33521) of the present invention and/or of the antimicrobial composition of the present invention.

It is contemplated that the pharmaceutical composition of the present invention comprises any suitable pharmaceutical carrier known in the state of the art. Said carrier must not unacceptably alter the activity of the strain *Lactococcus lactis* C11JN (Deposit number: DSM 33521) of the present invention and/or of the antimicrobial composition of the present invention.

Furthermore, the present invention in a fifth aspect, refers to a veterinary composition comprising *Lactococcus lactis* C11JN (Deposit number: DSM 33521) of the present invention, an antimicrobial composition of the present invention or combinations thereof.

*Lactococcus lactis* C11JN (Deposit number: DSM 33521) is as explained above in the first aspect of the present invention.

The antimicrobial composition of the present invention is as explained above in the second aspect of the present invention.

The veterinary composition of the present invention can be in any form known in the state of the art, preferably, the veterinary composition of the present invention is in solid or liquid form, more preferably in the form of a tablet, powder, solid lyophilized or liquid, more preferably solid lyophilized or liquid.

Preferably, the veterinary composition of the present invention is adapted or suitable for oral administration.

It is contemplated that the veterinary composition of the present invention comprises one or more further active compounds. Said one or more further active compounds can be any compound known in the state of the art. Preferably, said one or more active compounds are selected from antibiotics, more preferably β-lactam antibiotics, more preferably amoxicillin, cephalosporin or combinations thereof.

It is contemplated that the veterinary composition of the present invention comprises any suitable veterinary excipient known in the state of the art. Said excipients must not unacceptably alter the activity of the strain *Lactococcus lactis* C11JN (Deposit number: DSM 33521) of the present invention and/or of the antimicrobial composition of the present invention.

It is contemplated that the veterinary composition of the present invention comprises any suitable veterinary carrier known in the state of the art. Said carrier must not unacceptably alter the activity of the strain *Lactococcus lactis* C11JN (Deposit number: DSM 33521) of the present invention and/or of the antimicrobial composition of the present invention.

In a sixth aspect, the present invention refers to a probiotic composition comprising the strain *Lactococcus lactis* C11JN (Deposit number: DSM 33521) of the present invention.

The strain *Lactococcus lactis* C11JN (Deposit number: DSM 33521) is as explained above in the first aspect of the present invention.

In a seventh aspect, the present invention refers to a postbiotic composition comprising one or more compounds obtained from the strain *Lactococcus lactis* C11JN (Deposit number DSM 33521) or a combinations of the strain *Lactococcus lactis* C11JN (Deposit number: DSM 33521) of the present invention and one or more compounds obtained from the strain Lactococcus lactis C11JN (Deposit number DSM 33521).

The strain *Lactococcus lactis* C11JN (Deposit number: DSM 33521) is as explained above in the first aspect of the present invention.

The one or more compounds obtained from the strain *Lactococcus lactis* C11JN (Deposit number DSM 33521) are as explained above in the second aspect of the present invention.

In a preferred embodiment, the one or more compounds obtained from the strain *Lactococcus lactis* C11JN (Deposit number DSM 33521) are the fermented medium of strain *Lactococcus lactis* C11JN inactivated (Deposit number DSM 33521) of the present invention, as explained above.

In another preferred embodiment, the one or more compounds obtained from the strain *Lactococcus lactis* C11JN (Deposit number DSM 33521) are the culture supernatant of a culture of the strain Lactococcus lactis C11JN (Deposit number DSM 33521).

In a most preferred embodiment, the one or more compounds obtained from the strain *Lactococcus lactis* C11JN (Deposit number DSM 33521) the culture supernatant of a culture of the strain *Lactococcus lactis* C11JN (Deposit number DSM 33521) together with the *Lactococcus lactis* C11JN cells inactivated.

Preferably, the postbiotic composition of the present invention comprises one or more compounds obtained from the strain Lactococcus lactis C11JN (Deposit number DSM 33521) but not *Lactococcus lactis* C11JN (Deposit number DSM 33521), more preferably, the postbiotic composition only comprises as active ingredient the one or more compounds obtained from the strain Lactococcus lactis C11JN (Deposit number DSM 33521).

In an eighth aspect, the present invention refers to a *Lactococcus lactis* C11JN (Deposit number: DSM 33521) of the present invention, an antimicrobial composition of the present invention, a pharmaceutical composition of the present invention or a veterinary composition of the present invention for use as a medicament.

The *Lactococcus lactis* C11JN (Deposit number: DSM 33521) is as explained above in the first aspect of the present invention.

The antimicrobial composition of the present invention is as explained above in the second aspect of the present invention.

The pharmaceutical composition of the present invention is as explained above in the fourth aspect of the present invention.

The veterinary composition of the present invention is as explained above in the fifth aspect of the present invention.

The use as a medicament is in a subject in need of the treatment. Said subject in need of the treatment can be a human or an animal.

In an embodiment the subject in need of the treatment is a human.

In a preferred embodiment, the subject in need of the treatment is an animal, more preferably an animal selected from a ruminant, swine or a bird, even more preferably an animal selected from bovine, ovine, bird or wild boar.

In the eight aspect of the present invention, the *Lactococcus lactis* C11JN (Deposit number: DSM 33521), the antimicrobial composition of the present invention, the pharmaceutical composition of the present invention or the veterinary composition of the present invention is administered by any route known in the state of the art, more preferably, by means of oral administration.

In an ninth aspect, the present invention refers to a *Lactococcus lactis* C11JN (Deposit number: DSM 33521) of the present invention, an antimicrobial composition of the present invention, a pharmaceutical composition of the present invention or a veterinary composition of the present invention for use in the prevention, amelioration or treatment of a microbial disease.

The *Lactococcus lactis* C11JN (Deposit number: DSM 33521) is as explained above in the first aspect of the present invention.

The antimicrobial composition of the present invention is as explained above in the second aspect of the present invention.

The pharmaceutical composition of the present invention is as explained above in the fourth aspect of the present invention.

The veterinary composition of the present invention is as explained above in the fifth aspect of the present invention.

In this ninth aspect of the present invention the treatment is administered to a subject in need thereof. Said subject in need thereof can be a human or an animal.

In an embodiment the subject in need thereof is a human.

In a preferred embodiment, the subject in need thereof is an animal, more preferably an animal selected from a ruminant, swine or a bird, even more preferably an animal selected from bovine, ovine, bird or wild boar.

In the ninth aspect of the present invention, the *Lactococcus lactis* C11JN (Deposit number: DSM 33521), the antimicrobial composition of the present invention, the pharmaceutical composition of the present invention or the veterinary composition of the present invention is administered by any route known in the state of the art, more preferably, by means of oral administration.

In a preferred embodiment, the microbial disease is a bacterial disease; more preferably the bacterial disease is a disease produced by a bacteria of a genus selected from *Listeria*, *Streptococcus*, *Enterococcus*, *Salmonella*, *Escherichia*, *Manheimia*, *Pasterurella*, *Biberstenia* or *Clostridium*; more preferably the bacterial disease is a disease produced by *Listeria monocytogenes*, *Streptococcus suis*, *Enterococcus faecium*, *Salmonella typhimurium*, *Escherichia coli*, *Manheimia haemolytica*, *Pasterurella spp*., *Biberstenia trehalosi* or *Clostridium perfringens*; more preferably the bacterial disease is a disease produced by *Listeria monocytogenes*, *Streptococcus suis*, *Enterococcus faecium*, *Manheimia haemolytica* 813, *Pasterurella spp*., *Biberstenia trehalosi* or *Clostridium perfringens*; more preferably the bacterial disease is a disease produced by a gram-negative bacteria; more preferably, the bacterial disease is a disease produced by *Pasterurella spp.*, *Biberstenia trehalosi*; even more preferably, the bacterial disease is a disease produced by *Pasterurella spp.* 1315,2, *Pasteurella multocida A* 1269,2, *Pasteurella multocida B* 1291,1, *Pasteurella multocida D* 1277,4 or *Biberstenia trehalosi* 1315,1.

In a further preferred embodiment, the microbial disease is a bacterial disease selected from respiratory syndromes caused by bacteria of the genus Pasteurella or Manheimia, listeriosis, septicaemia, salmonellosis, colibacilosis, fibrinopurulent bronchopneumonia, pneumonic pasteurellosis, fibrinous pleuropneumonia, hemorrhagic septicaemia or enterotoxemia; more preferably, listeriosis, septicaemia, fibrinopurulent bronchopneumonia, pneumonic pasteurellosis, fibrinous pleuropneumonia, hemorrhagic septicaemia or enterotoxemia; even more preferably, fibrinopurulent bronchopneumonia, pneumonic pasteurellosis, fibrinous pleuropneumonia or hemorrhagic septicaemia.

In a most preferred embodiment, the subject in need of the treatment is a wild boar and the microbial disease is produced by *Listeria monocytogenes,* more preferably the microbial disease produced by *Listeria monocytogenes* is listeriosis.

In another most preferred embodiment, the subject in need of the treatment is a wild boar and the microbial disease is produced by *Streptococcus suis.*

In a further most preferred embodiment, the subject in need of the treatment is a bird and the microbial disease is produced by *Enterococcus faecium,* more preferably the microbial disease produced by *Enterococcus faecium* is septicaemia.

In an additional preferred embodiment, the subject in need of the treatment is a bird and the microbial disease is produced by *Salmonella typhimurium,* more preferably the microbial disease produced by *Salmonella typhimurium* is salmonellosis.

In a further preferred embodiment, the subject in need of the treatment is a calf and the microbial disease is produced by *Escherichia coli,* more preferably the microbial disease produced by *Escherichia coli* is colibacilosis.

In an additional most preferred embodiment, the subject in need of the treatment is bovine and the microbial disease is produced by *Manheimia haemolytica* 813, more preferably the microbial disease produced by *Manheimia haemolytica* 813 is fibrinopurulent bronchopneumonia.

In another most preferred embodiment, the subject in need of the treatment is ovine and the microbial disease is produced by *Pasterurella spp.* 1315,2, more preferably the microbial disease produced by *Pasterurella spp.* 1315,2 is pneumonic pasteurellosis.

In a further most preferred embodiment, the subject in need of the treatment is ovine and the microbial disease is produced by *Pasteurella multocida A* 1269,2*,* more preferably the microbial disease produced by *Pasteurella multocida A 1269,2* is fibrinous pleuropneumonia.

In an additional most preferred embodiment, the subject in need of the treatment is bovine and the microbial disease is produced by *Pasteurella multocida B* 1291,1, more preferably the microbial disease produced by *Pasteurella multocida B* 1291,1 is haemorrhagic septicaemia.

In a further most preferred embodiment, the subject in need of the treatment is ovine and the microbial disease is produced by *Pasteurella multocida D* 1277,4, more preferably the microbial disease produced by *Pasteurella multocida D* 1277,4 is pneumonic pasteurellosis.

In an additional most preferred embodiment, the subject in need of the treatment is ovine and the microbial disease is produced by *Biberstenia trehalosi* 1315,1, more preferably the microbial disease produced by *Biberstenia trehalosi* 1315,1 is pneumonic pasteurellosis.

In an additional most preferred embodiment, the subject in need of the treatment is bovine and the microbial disease is produced by *Clostridium perfringens,* more preferably the microbial disease produced by *Clostridium perfringens* is enterotoxemia.

In a tenth aspect, the present invention refers to the use of *Lactococcus lactis* C11JN (Deposit number: DSM 33521) of the present invention, an antimicrobial composition of the present invention, a pharmaceutical composition of the present invention or a veterinary composition of the present invention for the manufacture of a medicament for the prevention, amelioration or treatment of a microbial disease.

The *Lactococcus lactis* C11JN (Deposit number: DSM 33521) is as explained above in the first aspect of the present invention.

The antimicrobial composition of the present invention is as explained above in the second aspect of the present invention.

The pharmaceutical composition of the present invention is as explained above in the fourth aspect of the present invention.

The veterinary composition of the present invention is as explained above in the fifth aspect of the present invention.

The microbial disease is as explained above in the ninth aspect of the present invention.

In the tenth aspect of the present invention, the *Lactococcus lactis* C11JN (Deposit number: DSM 33521), the antimicrobial composition of the present invention, the pharmaceutical composition of the present invention or the veterinary composition of the present invention is administered by any route known in the state of the art, more preferably, by means of oral administration.

In a final aspect, the present invention refers to a method for the prevention, amelioration or treatment of a microbial disease comprising administering *Lactococcus lactis* C11JN (Deposit number: DSM 33521) of the present invention, an antimicrobial composition of the present invention, a pharmaceutical composition of the present invention or a veterinary composition of the present invention to a subject in need thereof.

The strain *Lactococcus lactis* C11JN (Deposit number: DSM 33521) is as explained above in the first aspect of the present invention.

The antimicrobial composition of the present invention is as explained above in the second aspect of the present invention.

The pharmaceutical composition of the present invention is as explained above in the fourth aspect of the present invention.

The veterinary composition of the present invention is as explained above in the fifth aspect of the present invention.

The microbial disease is as explained above in the ninth aspect of the present invention.

The subject in need thereof is as explained above in the ninth aspect of the present invention.

In this final aspect of the present invention, the *Lactococcus lactis* C11JN (Deposit number: DSM 33521), the antimicrobial composition of the present invention, the pharmaceutical composition of the present invention or the veterinary composition of the present invention is administered by any route known in the state of the art, more preferably, by means of oral administration.

### REFERENCE TO DEPOSITED BIOLOGICAL MATERIAL

The microorganism object of the present invention, *Lactococcus lactis* C11JN, has been deposited on May 8^{th}, 2020 by INNOVACION EN GESTION Y CONSERVACION DE UNGULADOS S.L. (INGULADOS) in the Leibniz Institute DSMZ-German Collection of Microorganisms and Cell Cultures with the Deposit number DSM 33521.

### SEQUENCE LISTING FREE TEXT

SEQ ID NO: 1
   <223> Genome of the strain *Lactococcus lactis* C11JN (Deposit number: DSM 33521)
   <223> These positions can be a, t, g or c
   <223> These positions can be a, t, g or c
   <223> These positions can be a, t, g or c
   <223> These positions can be a, t, g or c
   <223> These positions can be a, t, g or c
   <223> These positions can be a, t, g or c
   <223> These positions can be a, t, g or c
   <223> These positions can be a, t, g or c
SEQ ID NO: 2
   <223> Positions 1864240 to 1864341 of SEQ ID NO: 1, coding sequence for Nisin A, Nisin Z, Nisin Q and Nisin F
SEQ ID NO: 3
   <223> Nisin A in *Lactococcus lactis* C11JN (Deposit number DSM 33521)
SEQ ID NO: 4
   <223> Nisin Z in Lactococcus lactis C11JN (Deposit number DSM 33521)
SEQ ID NO: 5
   <223> Nisin Q in Lactococcus lactis C11JN (Deposit number DSM 33521)
SEQ ID NO: 6
   <223> Nisin F in Lactococcus lactis C11JN (Deposit number DSM 33521)
SEQ ID NO: 7
   <223> Positions 1864240 to 1864329 of SEQ ID NO: 1, coding sequence for Nisin U
SEQ ID NO: 8
   <223> Nisin U in Lactococcus lactis C11JN (Deposit number DSM 33521)
SEQ ID NO: 9
   <223> Positions 29295 to 29723 of SEQ ID NO: 1, coding sequence for Enterolysin A
SEQ ID NO: 10
   <223> Enterolysin A in Lactococcus lactis C11JN (Deposit number DSM 33521)
SEQ ID NO: 11
   <223> Positions 1864246 to 1864329 of SEQ ID NO: 1, coding sequence for Subtilin
SEQ ID NO: 12
   <223> Subtilin in Lactococcus lactis C11JN (Deposit number DSM 33521)
SEQ ID NO: 13
   <223> Positions 613895 to 614665 of SEQ ID NO: 1, coding sequence for Zoocin A
SEQ ID NO: 14
   <223> Zoocin A in Lactococcus lactis C11JN (Deposit number DSM 33521)

To allow a better understanding, the present invention is described in more detail below with reference to the enclosed figure, which is presented by way of example, and with reference to illustrative and non-limitative examples.

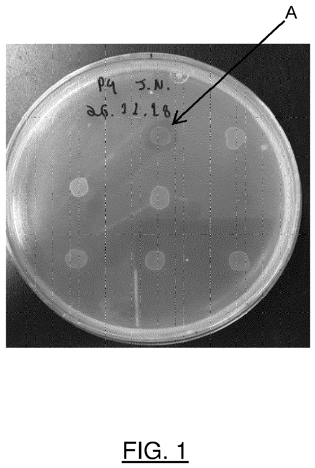

Figure 1 shows a photography of a Mueller-Hinton medium agar plate in which the antimicrobial droplet assay was performed in accordance with example 1 included below. Noted with an A is the colony of *Lactococcus lactis* C11JN (Deposit number: DSM 33521), which, as can be readily seen is the one that provided the biggest halo of inhibition of growth of the pathogenic bacteria (*Streptococcus suis*).

### Example 1. Isolation, identification and characterization of the strain Lactococcus lactis C11JN (Deposit number: DSM 33521).

A wild boar population from a hunting farm was selected for the sampling procedure. All animals presented an optimal sanitary state with low prevalence of diseases. Samples from nasal mucosa were collected from young animals, using Amies transport medium swab (DELTALAB, Barcelona Spain) kept in refrigeration until processing in the laboratory.

For the Lactic Acid Bacteria (hereinafter, LAB) isolation, swabs were placed in saline buffer for serial dilutions. 100 µL of each dilution were plated onto MRS Agar medium (Condalab, Madrid, Spain) for the isolation of LAB and on Plate Count Agar (PCA; composition: 5 g/L of enzymatic digest of casein, 2.5 g/mL of yeast extract, 1 g/L of glucose and 15 g/L of bacteriological agar) to count total mesophilic aerobes. All plates were incubated at 37 °C for 24-48 h in aerobiosis. Subsequently, individual colonies from LAB specific medium were propagated in microdilution plates with broth medium (MRS Agar medium mentioned above). Microdilution plates were incubated 24 h at 37 °C in aerobiosis.

To select the strains of lactic acid bacteria with antimicrobial activity, the Cell To Cell Competition method was performed. This method consists of testing the activity of isolated LAB with a pathogenic indicator bacterium. In this case, the first screening was implemented with a *Streptococcus suis* indicator strain isolated from a clinical case of a wild boar.

A solution of the pathogenic bacteria (*Streptococcus suis*) with a turbidity of 0.5 McFarland was prepared from an overnight culture and was plated onto Mueller-Hinton medium (hereinafter, MH) agar. Subsequently ADT (antimicrobial droplet assay) method was implemented by placing 5 µL from each broth cultures in the MH plates previously inoculated with the pathogenic bacteria. The plates were incubated between 24 and 48 h, and the strains that produced a halo of inhibition of the pathogenic bacteria during their growth were selected (Figure 1).

This strain (only one strain was found to produce was found to produce halo of inhibition in all samples) re-seeded in liquid and solid MH and it was identified using staining morphologic, biochemical and molecular characteristics.

The isolated bacterial strain that was identified as C11JN was a Gram-positive coccus (cells are spherical or ovoid and occur singly, in pairs, or in chains, and are often elongated in the direction of the chain), catalase negative. Its genomic DNA was extracted with the SpeedTools Tissue DNA Extraction Kit (Biotools, Madrid, Spain). The entire genome was sequenced generating a 2,080 Mbp sample (SEQ ID NO: 1). Analysis of similar sequences in the GenBank database resulted in 99.16 % identity with the *Lactococcus lactis* subsp. lactis 111403 chromosome (GenBank, NC_002662.1, as consulted on May 12^{th}, 2020). This analysis confirmed that it is a non-pathogenic bacterium (Generally Recognized as Safe, GRAS) whose antimicrobial activity may be due to the production of bacteriocins (for example, nisin).

Further analysis confirmed the following features of *Lactococcus lactis* C11JN strain:
- Cells do not form endospores.
- Cells are nonmotile.
- Cells are not β-hemolytic.
- Cells are aerobic.

Bioinformatic analysis of the genome of the *Lactococcus lactis* C11JN strain was carried out to compare protein sequences to bacteriocins using the BACTIBASE and BAGEL4 databases and homology analysis. The following bacteriocins were identified in the genome with homologies from 40 to 70% with other ones reported in the databases:
- Nisins:
   - Nisin A (SEQ ID NO: 3), which would correspond to positions 1864240 to 1864341 of SEQ ID NO: 1 (SEQ ID NO: 2);
   - Nisin Z (SEQ ID NO: 4), which would correspond to positions 1864240 to 1864341 of SEQ ID NO: 1 (SEQ ID NO: 2);
   - Nisin Q (SEQ ID NO: 5), which would correspond to positions 1864240 to 1864341 of SEQ ID NO: 1 (SEQ ID NO: 2);
   - Nisin F (SEQ ID NO: 6), which would correspond to positions 1864240 to 1864341 of SEQ ID NO: 1 (SEQ ID NO: 2);
   - Nisin U (SEQ ID NO: 8), which would correspond to positions 1864240 to 1864329 of SEQ ID NO: 1 (SEQ ID NO: 7).
- Enterolysin A (SEQ ID NO: 10), which would correspond to positions 29295 to 29723 of SEQ ID NO: 1 (SEQ ID NO: 9).
- Subtilin (SEQ ID NO: 12), which would correspond to positions 1864246 to 1864329 of SEQ ID NO: 1 (SEQ ID NO: 11).
- Zoocin A (SEQ ID NO: 14), which would correspond to positions 613895 to 614665 of SEQ ID NO: 1 (SEQ ID NO: 13).

Bacteriocins, as it is commonly known in the state of the art, are low molecular weight peptides secreted by bacteria whose function is to inhibit the growth of other bacteria present in the same ecosystem as the secretory bacteria in which they compete for nutrients. Most bacteriocins have antibacterial properties, and some of them also antiviral or antifungal. Bacteriocins produced by Gram positive bacteria, especially LAB, are considered safe and are used in industrial applications, being nisin the only purified bacteriocin whose food application is approved by the FDA (United States Food and Drug Administration). The new *Lactococcus lactis* C11JN strain identified herein secretes up to 5 types of nisin, in addition to other bacteriocins.

Therefore, the new *Lactococcus lactis* C11JN strain provides for a new antimicrobial product with a broad-spectrum activity, which can be used as a probiotic composition (by administering directly the strain), as a postbiotic composition or as a composition comprising the bioactive compounds generated by the new *Lactococcus lactis* C11JN strain.

### Example 2. Determination of the antimicrobial activity of the strain Lactococcus lactis C11JN (Deposit number: DSM 33521) with regard to several animal pathogens by means of the microdilution method (postbiotic activity)

The tests to determine the antimicrobial activity of the *Lactococcus lactis* C11JN strain were carried out against bacterial pathogenic strains isolated from clinical cases in animals (see details of the bacterial species below in Table 1). Many of pathogenic bacteria used in the present example are zoonotic, and comprise both Gram-positive and Gram-negative bacteria, although according to the literature, the activity of the bacteriocins secreted by LAB usually have an effect against Gram-positive bacteria.

Through these techniques, the postbiotic activity of the bioactive compounds secreted by the bacteria during its growth was determined, including bacteriocins, but excluding bacterial cells. Culture conditions for the strain *Lactococcus lactis* C11JN were optimized to obtain the highest protein compounds production. Two different culture media (De Mann Rogosa and Sharpe, hereinafter MRS and Tryptone Soy Broth, hereinafter TSB) and two growth temperatures (30 and 37 °C) were tested and Bradford method was used for proteins quantification. The optimal conditions were found to be: MRS, medium at 30 °C, aerobiosis.

The supernatants of the different culture conditions of the *Lactococcus lactis* C11JN strain were obtained by centrifuging the broth culture at 6,000 rpm for 10 minutes followed by filtering with 0.22 µm millipore filters. Finally, an inactivation step was conducted in a thermoblock at 100 °C for 10 minutes.

Antimicrobial activity was tested using the Minimum Inhibitory Concentration (hereinafter, MIC), one of the most widely used antimicrobial sensitivity testing methods. The procedure involved the preparation of dilutions of the antimicrobial agent (in the present case, the different supernatants obtained from the *Lactococcus lactis* C11JN strain) in a broth growth medium using a 96-well microplate. Each well was then inoculated with a pathogen prepared in the same medium and the microplate was incubated under suitable conditions (for the growth of the pathogen) depending on the test pathogen.

The activity of the supernatants against the different pathogens was measured in Activity Units per millilitre (AU/mL) which is the reciprocal of the highest dilution showing inhibition of the growth indicator (this is, absence of turbidity). The absence of growth was verified by massive plating onto MH plate. For example, a growth inhibition at dilution 1/2 corresponds to 2,000 AU/mL.

The results obtained for the *Lactococcus lactis* C11JN supernatant activities under optimal culture conditions (MRS, medium at 30 °C, aerobiosis) were as follows (Table 1):

**Table 1. Details of the pathogenic bacteria strains tested in example 2, their origin species and disease as well as the results of antimicrobial activity obtained.**

| **Bacterial pathogenic strain** | **Origin species y and disease** | ***Lactococcus lactis* C11JN antimicrobial activity (AU/ml)** |
|---|---|---|
| *Listeria monocytogenes* | Wild boar, listeriosis | 4,000 |
| *Streptococcus suis* | Wild boar | 4,000 |
| *Enterococcus faecium* | Bird, septicaemia | 4,000 |
| *Salmonella typhimurium* | Bird, salmonellosis | 2,000 |
| *Escherichia coli* | Calf, colibacilosis | 1,000 |
| *Manheimia haemolytica 813* | Bovine, fibrinopurulent bronchopneumonia | 4,000 |
| *Pasterurella spp. 1315,2* | Ovine, pneumonic pasteurellosis | 8,000 |
| *Pasteurella multocida A 1269,2* | Ovine, fibrinous pleuropneumonia | 8,000 |
| *Pasteurella multocida B 1291,1* | Bovine, haemorrhagic septicaemia | 8,000 |
| *Pasteurella multocida D* 1277,4 | Ovine, pneumonic pasteurellosis | 8,000 |
| *Biberstenia trehalosi 1315,1* | Ovine, pneumonic pasteurellosis | 8,000 |
| *Clostridium perfringens* | Bovine, enterotoxemia | 4,000 |

As can be directly derived from table 1, the new *Lactococcus lactis* C11JN strain showed activity against all the bacterial pathogens and diseases tested. It is relevant to highlight that there is a group of bacterial pathogens against which the present invention showed an increased activity (see bacterial pathogens and diseases in which the antimicrobial activity is 4,000 UA/ml or higher).

Therefore, the new *Lactococcus lactis* C11JN strain and the bioactive compounds produced by said strain are effective against a broad-spectrum of bacteria, including both gram-positive and gram-negative bacteria, obtained from different animal species and causing different diseases. Hence, the new *Lactococcus lactis* C11JN strain provides for a new antimicrobial product with a broad-spectrum activity, which can be used as a probiotic composition (by administering directly the strain), as a postbiotic composition, as a pharmaceutical composition, as a veterinary composition or as a composition comprising the bioactive compounds generated by the new *Lactococcus lactis* C11JN strain.

### Example 3. Antimicrobial activity of the strain Lactococcus lactis C11JN (Deposit number: DSM 33521) with regard to several animal pathogens by means the coculture method (postbiotic activity)

In this example, the antimicrobial activity of the *Lactococcus lactis* C11JN strain against the bacterial pathogens which showed the greatest supernatants activities in example 2, was analysed using the co-culture technique (see details of the bacterial species below in Table 2). This technique has traditionally been used to identify metabolites with antimicrobial activity and consists of incubating the indicator bacterial pathogenic strain and the bacterium with potential antimicrobial activity under the same conditions, to determine a possible inhibition of the pathogen growth.

The protocol consisted of culturing 20 µL of an overnight culture at optimal conditions of the bacterial pathogenic indicator strain and the strain with potential antimicrobial activity (in the present case, *Lactococcus lactis* C11JN) in a 20 mL non-selective MH medium coculture. The negative control monoculture control was prepared by adding 20 µL of the overnight culture of the bacterial pathogenic strain in 20 mL of MH. Monoculture controls and co-cultures were incubated at 37 °C in aerobiosis for 24 h.

Subsequently, serial microdilution was performed in 96-well plates of each of the samples and controls plated in solid medium (Blood Agar) to perform counts of CFU (colony forming units)/ml, in the present case up to the 10⁻⁶ dilution, during 24/48 h in aerobiosis at 37 °C. The differentiation of the colonies between the bacterial pathogenic and the *Lactococcus lactis* C11JN strains was performed by means of the morphology of the colonies and gram staining characteristics.

The results obtained in this example are expressed as a percentage of growth reduction of the bacterial pathogenic strain and are shown in Table 2.

**Table 2. Shows the results obtained in example 3 with regard to the growth of the tested pathogenic bacteria, both for the controls and for the cocultures of the pathogenic bacteria and Lactococcus lactis C1 1JN**

| **Bacterial pathogenic strain** | **Counts of CFU/ml the control Bacterial pathogenic strain monoculture** | **Counts of CFU/ml of the bacterial pathogenic strain coculture** | **% reduction of bacterial pathogenic strain growth** |
|---|---|---|---|
| *Pasterurella* spp. | 2.1x10⁶ | 1x10⁵ | 95.24 % |
| *Manheimia haemolytica* | 2.34x10⁷ | 5x10⁵ | 97.87 % |

The results show that the *Lactococcus lactis* C11JN strain is capable of producing certain metabolites or bioactive compounds during its growth that have antimicrobial activity against gram negative bacteria, such as *Pasteurella* and *Manheimia.* This fact is unique and novel since the antibacterial activity of bacteriocins secreted by lactic acid bacteria in the state of the art have been generally associated with antimicrobial activity against gram positive pathogens.

These results (together with those of examples 1 and 2 included above), validate the fact that the new *Lactococcus lactis* C11JN strain provides for a new antimicrobial product with a broad-spectrum activity effective both against gram-positive and gram-negative bacteria, which can be used as a probiotic composition (by administering directly the strain), as a postbiotic composition, as a pharmaceutical composition, as a veterinary composition or as a composition comprising the bioactive compounds generated by the new *Lactococcus lactis* C11JN strain.

## Claims

1. A strain of *Lactococcus lactis* termed *Lactococcus lactis* C11JN (Deposit number: DSM 33521).

2. Antimicrobial composition antimicrobial composition comprising *Lactococcus lactis* C11JN (Deposit number DSM 33521) in accordance with claim 1, one or more compounds obtained from the strain *Lactococcus lactis* C11JN (Deposit number DSM 33521) in accordance with claim 1 or combinations thereof.

3. Antimicrobial composition in accordance with claim 2 **characterized in that** the one or more compounds obtained from the strain *Lactococcus lactis* C11JN (Deposit number DSM 33521) is the fermented medium of said strain.

4. Antimicrobial composition in accordance with claim 2 or claim 3 **characterized in that** the one or more compounds obtained from the strain *Lactococcus lactis* C11JN (Deposit number DSM 33521) is the culture supernatant of said strain.

5. Antimicrobial composition in accordance with any one of claims 2 to 4, **characterized in that** the one or more compounds obtained from the strain *Lactococcus lactis* C11JN (Deposit number DSM 33521) is obtained by means of a procedure comprising the following steps:
a) collecting the culture supernatant of a culture of a strain *Lactococcus lactis* C11JN (Deposit number DSM 33521) in accordance with claim 1;
b) centrifuging said supernatant to obtain a centrifuged supernatant;
c) filtering the centrifuged supernatant;
d) inactivating the solution obtained in step c) by means of heat treatment.

6. Antimicrobial composition in accordance with any one of claims 2 to 5, **characterized in that** the one or more compounds obtained from the strain *Lactococcus lactis* C11JN (Deposit number DSM 33521) comprise: at least one type of Nisin, Enterolysin A, Subtilin, Zoocin A or combinations thereof.

7. Food composition **characterized in that** it comprises *Lactococcus lactis* C11JN (Deposit number DSM 33521) in accordance with claim 1, an antimicrobial composition in accordance with any one of claims 2 to 6 or combinations thereof.

8. A food composition in accordance with claim 7, **characterized in that** it is a food supplement, a nutraceutical, a probiotic, a symbiotic or a postbiotic.

9. Pharmaceutical composition **characterized in that** it comprises *Lactococcus lactis* C11JN (Deposit number DSM 33521) in accordance with claim 1, an antimicrobial composition in accordance with any one of claims 2 to 6 or combinations thereof.

10. Veterinary composition **characterized in that** it comprises *Lactococcus lactis* C11JN (Deposit number DSM 33521) in accordance with claim 1, an antimicrobial composition in accordance with any one of claims 2 to 6 or combinations thereof.

11. *Lactococcus lactis* C11JN (Deposit number DSM 33521) strain in accordance with claim 1, antimicrobial composition in accordance with claims 2 to 6, pharmaceutical composition in accordance with clam 9 or veterinary composition in accordance with claim 10 for use as a medicament.

12. *Lactococcus lactis* C11JN (Deposit number DSM 33521) strain in accordance with claim 1, antimicrobial composition in accordance with claims 2 to 6, pharmaceutical composition in accordance with clam 9 or veterinary composition in accordance with claim 10 for use in the prevention, amelioration or treatment of a microbial disease.

13. *Lactococcus lactis* C11JN (Deposit number DSM 33521) strain in accordance with claim 1, antimicrobial composition in accordance with claims 2 to 6, pharmaceutical composition in accordance with clam 9 or veterinary composition in accordance with claim 10 for use in accordance with claim 12, **characterized in that** the microbial disease is a bacterial disease.

14. *Lactococcus lactis* C11JN (Deposit number DSM 33521) strain in accordance with claim 1, antimicrobial composition in accordance with claims 2 to 6, pharmaceutical composition in accordance with clam 9 or veterinary composition in accordance with claim 10 for use in accordance with claim 13, **characterized in that** the bacterial disease is a disease produced by *Listeria monocytogenes, Streptococcus suis, Enterococcus faecium, Salmonella typhimurium, Escherichia coli, Manheimia haemolytica 813, Pasterurella spp. 1315,2, Pasteurella multocida A 1269,2, Pasteurella multocida B 1291,1, Pasteurella multocida D 1277,4, Biberstenia trehalosi 1315,1 or Clostridium perfringens.*

15. *Lactococcus lactis* C11JN (Deposit number DSM 33521) strain in accordance with claim 1, antimicrobial composition in accordance with claims 2 to 6, pharmaceutical composition in accordance with clam 9 or veterinary composition in accordance with claim 10 for use in accordance with claim 13, **characterized in that** the bacterial disease is selected from respiratory syndromes caused by bacteria of the genus Pasteurella or Manheimia, listeriosis, septicaemia, salmonellosis, colibacilosis, fibrinopurulent bronchopneumonia, pneumonic pasteurellosis, fibrinous pleuropneumonia, hemorrhagic septicaemia or enterotoxemia.
